**Europäisches Patentamt**

**European Patent Office**

**Office européen des brevets**

(19)

(11) Publication number: **0 135 022**
**A2**

(12) # EUROPEAN PATENT APPLICATION

(21) Application number: **84108344.7**

(22) Date of filing: **16.07.84**

(51) Int. Cl.⁴: **A 61 K 39/35**
**A 61 K 9/26**

(30) Priority: **27.07.83 GB 8321178**
**05.11.83 GB 8329619**

(43) Date of publication of application:
**27.03.85 Bulletin 85/13**

(84) Designated Contracting States:
**BE CH DE FR GB IT LI NL SE**

(71) Applicant: **BEECHAM GROUP PLC**
**Beecham House Great West Road**
**Brentford Middlesex TW8 9BD(GB)**

(72) Inventor: **Moran, David Martin**
**1 Durnsford Way**
**Cranleigh Surrey(GB)**

(72) Inventor: **Vezin, William Roger**
**61 Castle Road**
**Hadleigh Ipswich Suffolk(GB)**

(74) Representative: **Russell, Brian John et al,**
**European Patent Attorney Beecham Pharmaceuticals**
**Great Burgh Yew Tree Bottom Road**
**Epsom Surrey, KT18 5XQ(GB)**

(54) Allergen-containing composition.

(57) A composition comprising a dispersion of allergen particles in a matrix of alkali or water soluble, acid insoluble polymer, and its use in the therapy of allergic humans.

EP 0 135 022 A2

TITLE MODIFIED
see front page

## COMPOSITIONS

This invention relates to compositions containing allergens and their use in the therapy of allergic humans.

Many people elicit characteristic respiratory symptoms to common aeroallergen materials such as pollens and house dust. Such allergic symptoms have been conventionally treated by the administration to the sufferer of repeated, gradually increasing doses of the relevant allergen to build up resistance to the allergen.

Oral administration of aqueous allergen vaccines has been employed for many decades, particularly for use with children. Although the mechanisms of action of such vaccines remain uncertain, there are several disadvantages in their use. Firstly, the allergenic proteins can be degraded rapidly by gastric secretions, thereby reducing the access of the proteins to lymphoid tissue in the lower gut. Secondly, possible adverse side effects can result from allergenic exposure to buccal, oesophageal and tracheal tissue.

A novel allergen composition has now been developed which reduces or overcomes the above disadvantages.

According to the present invention there is provided a composition comprising a dispersion of allergen particles in a matrix of alkali or water soluble, acid insoluble polymer.

The invention also provides a pharmaceutical composition comprising a pharmaceutically acceptable form of the dispersion optionally in combination with a pharmaceutically acceptable carrier.

Suitable polymers for use in the composition of the invention include hydroxy propyl methyl cellulose, hydroxy propyl methyl cellulose phthalate, cellulose acetate phthalate, polyvinyl acetate phthalate, or a methacrylate or acrylate copolymer. Preferred polymers are methacrylic acid/methyl methacrylate copolymers such as Eudragit L or Eudragit S (Eudragit is a trade mark of Rohm and Haas).

The dispersion is preferably in the form of particles of a particle size from 100 μm to 1000μm, particularly from 200 μm to 500 μm, and these contain allergen particles which are preferably from 1 to 5 μm in diameter. The weight ratio of allergen to polymer in the dispersion can vary within wide limits, but a preferred ratio is from 0.01 to 10%w/w (allergen:polymer). The allergen particles are preferably in the form of an extract of allergen, and suitable allergens from which the extract can be obtained include pollens, such as grass pollens; weeds, such as ragweed; house dust mites and venoms, such as bee venom, and other allergens of animal origin such as hair, fur and dander.

Typically, animals which provide the aforementioned other allergens of animal origin are domesticated animals such as cats, dogs and horses.

The pharmaceutical compositions of the present invention are adapted for oral administration and particularly suitable compositions are unit dosage forms such as tablets and capsules. Other fixed unit dosage forms, such as powders presented in sachets, may also be used.

In accordance with conventional pharmaceutical practice a carrier may be used which comprises a diluent, filler, disintegrant, wetting agent, lubricant, colourant, flavourant or the like. Alternatively, a carrier may be absent from the composition, if desired.

Typical carriers may, therefore, comprise such agents as microcrystalline cellulose, starch, sodium starch glycollate, polyvinylpyrrolidone, polyvinyl-polypyrrolidone, magnesium stearate, sodium lauryl sulphate, sucrose and the like.

Most suitably the pharmaceutical composition will be provided in unit dose form.

From the above discussion it will be appreciated that the pharmaceutical composition of the invention has the advantage of being partially protected from the degradative effects of acid/pepsin environments for extended periods, while allowing rapid release of the allergen material at approximately neutral pH.

- 4 -

0135022

The dispersion of allergen particles in the polymer matrix may be prepared by combining a suspension of allergen particles in a liquid carrier with the polymer, preferably in powder form, so that the polymer dissolves in the carrier, removing the carrier and comminuting the solid residue to obtain the required particle size. Alternatively, an aqueous solution of allergen may be added to a pre-prepared solution of polymer in a liquid carrier, and the total liquid content removed to obtain the solid residue. The dispersion may then be incorporated into a pharmaceutical composition in accordance with conventional methods.

Suitable liquid carriers are $C_{1-4}$ alkanols.

A preferred liquid carrier is ethanol, optionally diluted with water, which may be removed by evaporation, and the comminuted residue may be sieved to obtain the required particle size range.

The following Examples illustrate the invention.

EXAMPLE 1

Preparation of mixed grass pollen extract/Eudragit L dispersion.

Mixed grass pollen extract was dissolved in phosphate-buffered saline to obtain a clear solution of final total protein concentration of ca. 2% w/v. (2-5%).

After filtering with a 0.45µm millipore filter sufficient aqueous solution was added to absolute ethanol to produce a final alcoholic suspension of 1 in $1 \times 10^4$ w/v.

The alcoholic suspension was added immediately to Eudragit L, dry powder grade (10 ml suspension to 1 g. of polymer powder) and stirred intermittently until the polymer was dissolved.

The suspension was placed in an ultrasonic bath for 5 minutes and films were cast by pouring the alcoholic polymer solution/protein suspension onto levelled flat glass troughs (float plate glass), ca. 1 g. polymer, 10 ml. solution per 60 $cm^2$ (20 - 100 $cm^2$).

The films were dried in a vacuum oven at 20° (15 - 25°c.) at 160 mm (100 - 200 mm) or at the minimum pressure at which bubbles do not form in the film. After two hours, the chamber was fully evacuated and left 24 hours, or until the films were brittle and easily removed.

The films were comminuted by mortar and pestle avoiding 'fines' in which protection is reduced, and the resultant material was sieved to obtain the particle size range of 100 - 1000 µm.

By replacing mixed grass pollen extract with house dust mite extract, the above procedure was used to prepare a house dust mite/Eudragit L dispersion.

## EXAMPLE 2

Absolute ethanol was added to powdered Eudragit L100 in the ratio 5ml ethanol to 1g Eudragit L100 and stirred with a magnetic stirrer at 20-25°C. For either at least 12 hours longer than the time required to obtain a visibly homogeneous solution, or 24 hours, whichever is the longer.

Mixed grass pollen dried extract was dissolved in phosphate buffered saline to obtain a clear solution of final total protein concentration of 2% w/v.

The aqueous pollen solution was filtered with a 0.45μ millipore filter and sufficient aqueous filtered solution was added to the ethanolic Eudragit L while stirring at 20-25°C, over a period of 1 minute, to produce a final protein polymer mass ratio of 1:1000.

Films were cast immediately by pouring the alcoholic polymer protein suspension onto levelled flat glass troughs (float plate glass) ca. 1g polymer (= 6ml solution) per 60cm2.

The films were dried in a vacuum oven at 20°C (15-25°C) at 160mm or at the minimum pressure at which bubbles do not form in the film. After two hours, the chamber was fully evacuated (<1 torr) for 24 hours, or until the films became brittle and easily removed.

The films were comminuted by hammer mill or mortar and pestle avoiding 'fines' in which protection is reduced, and the resulting material was sieved to obtain the particle size range 100-1000μm.

## BIOLOGICAL DATA

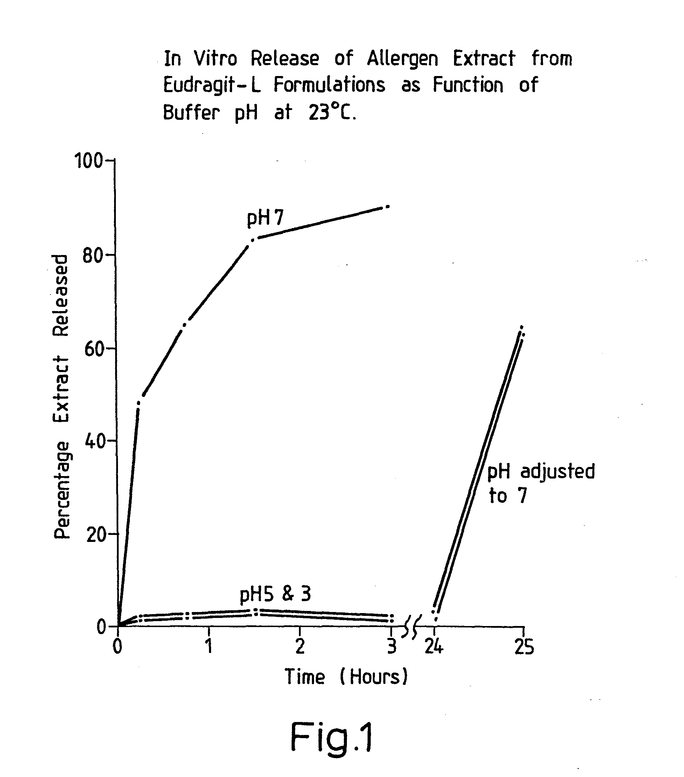

Figure 1 shows typical bioavailability release characteristics of mixed grass from Eudragit-L/mixed grass formulations (10:1 w/w) as a function of pH.

At acid pH, the allergen extract remains sequestered, although rapid release of material is achieved at neutral pH even after protracted exposure to high acidity buffers.

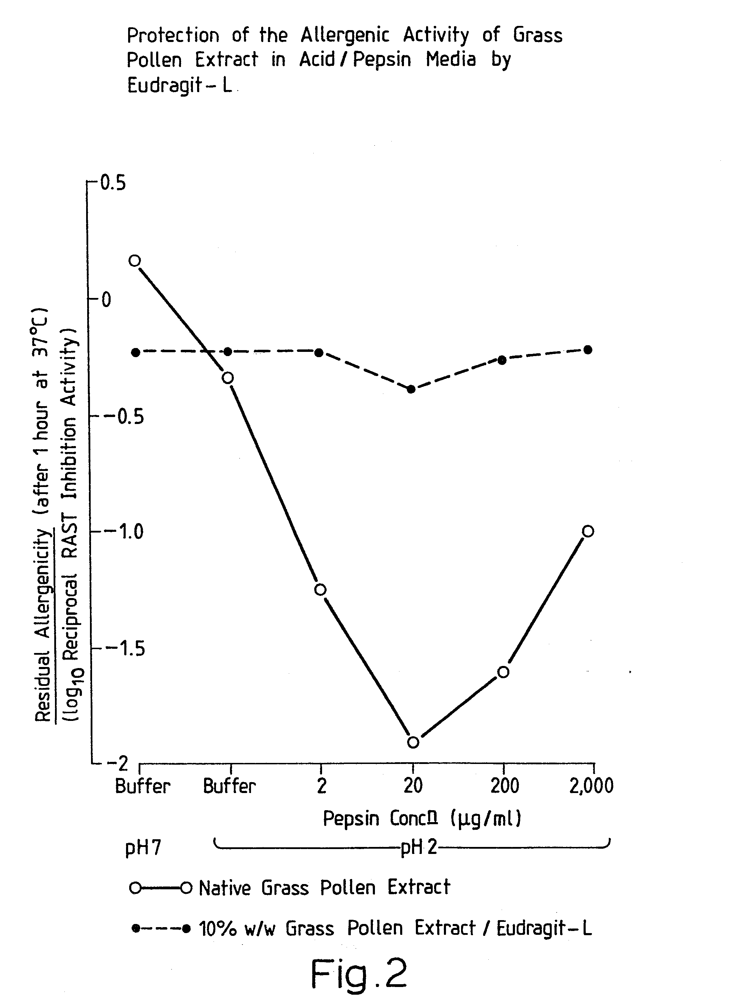

Figure 2 shows protection of the allergenicity of mixed grass extract against the degradative influence of acid/pepsin environments.

Table 1 shows the recovery of allergenic activity (measured by RAST inhibition) of 5 batches of Eudragit-L/mixed grass extract (10:1 w/w) and 2 batches of Eudragit-L) D. Pteronyssinus extract (10:1 w/w).

As is evident, with the exception of one batch of grass extract (i.e. No.5), a substantial recovery of the nominal allergenic activity is evident.

_____

## TABLE 1

Recovery of the Allergenic Activity of Eudragit-L Mixed
Grass and House Dust Mite Formulations.

| Formulation* | Batch No. | % Residual Allergenicity (RAST Inhibition) |
|---|---|---|
| Mixed Grass | 1 | 53 |
| Mixed Grass | 2 | 32 |
| Mixed Grass | 3a+ | 83 |
| Mixed Grass | 4b+ | 77 |
| Mixed Grass | 5 | 8 |
| D.pteronyssinus | 6a+ | 37 |
| D.pteronyssinus | 7b+ | 100 |

* All formulations contained 10% w/w extract

+ Suffix a or b represents sieve cuts  <70 mesh and
  50-70 mesh respectively.

0135022

Claims:

1. A composition comprising a dispersion of allergen particles in a matrix of alkali or water soluble, acid insoluble polymer.

2. A pharmaceutical composition comprising a pharmaceutically acceptable form of the dispersion claimed in claim 1 optionally in combination with a pharmaceutically acceptable carrier.

3. A composition as claimed in claim 1 or claim 2, wherein the polymer is hydroxy propyl methyl cellulose, hydroxy propyl methyl cellulose phthalate, cellulose acetate phthalate, polyvinyl acetate phthalate, or a methacrylate or acrylate copolymer.

4. A composition as claimed in any one of claims 1 to 3, wherein the polymer is a methacrylic acid/methyl methacrylate copolymer.

5. A composition as claimed in any one of claims 1 to 4, wherein the dispersion is in the form of particles of a particle size from 100μm to 1000μm.

6. A composition as claimed in any one of claims 1 to 5, wherein the weight ratio of allergen to polymer in the dispersion is from 0.01 to 10% w/w.

7. A composition as claimed in any one of claims 1 to 6, wherein allergens include pollens such as grass pollens; weeds such as ragweed; house dust mites, venoms such as bee venom and other allergens of animal origin such as hair, fur and dander.

8. A composition as claimed in any one of claims 1 to 7, wherein the allergen particles are in the form of an extract of allergen.

9. A composition as claimed in any one of claims 2 to 8, adapted for oral administration.

10. A composition as claimed in any one of claims 2 to 9, wherein the optional carrier comprises microcrystalline cellulose, starch, sodium starch glycollate, polyvinylpyrrolidone, polyvinyl-polypyrrolidone,magnesium stearate, sodium lauryl sulphate or sucrose.

In Vitro Release of Allergen Extract from Eudragit-L Formulations as Function of Buffer pH at 23°C.

Fig.1

Protection of the Allergenic Activity of Grass
Pollen Extract in Acid / Pepsin Media by
Eudragit – L

Fig.2